# EUROPEAN PATENT SPECIFICATION

(11) **EP 4 424 331 B1**
(45) Date of publication and mention of the grant of the patent: **21.10.2026**
(21) Application number: 24159601.4
(22) Date of filing: 26.02.2024
(51) Int. Cl.: A61L 9/014, A61L 9/015, A61L 9/14, A61L 9/20, A61L 9/22, B01D 46/00, F24F 8/22

(54) **DEVICE FOR AIR PURIFICATION AND SANITIZATION**
VORRICHTUNG ZUR LUFTREINIGUNG UND -DESINFEKTION
DISPOSITIF DE PURIFICATION ET DE DÉSINFECTION DE L'AIR

(30) Priority: 28.02.2023 IT 202300003618
(43) Date of publication of application: 04.09.2024
(73) Proprietor: Di Mauro, Concetto, 95045 Misterbianco (MT) (IT)
(72) Inventor: Di Mauro, Concetto, 95045 Misterbianco (MT) (IT)
(74) Representative: Lunati & Mazzoni S.r.L.

(56) References cited:
- EP-A1- 4 088 753
- US-A1- 2020 094 179
- NONAMI T ET AL: "Apatite-coated titanium dioxide photocatalyst for air purification", CATALYSIS TODAY, ELSEVIER, AMSTERDAM, NL, vol. 96, no. 3, 5 October 2004 (2004-10-05), pages 113 - 118, XP004570856, ISSN: 0920-5861, DOI: 10.1016/J.CATTOD.2004.06.112

## Description

The present invention relates to a device for purification and sanitization according to claim 1.

In particular, the present invention relates to a device for air or surfaces purification and sanitization that can be associated with a forced mechanical ventilation system, also known by the acronym FMV (Forced Mechanical Ventilation).

As is known, air purification and sanitization devices, also called air purifiers, are electrical appliances designed to allow the filtering and cleaning of the air in environments where, for example, there may be unpleasant odors such as smoke that create health problems for people, or especially for the elimination of germs and bacteria US 2020/094179 A1 and EP 4 088 753 A1 disclose air purification devices.

Generally, such devices can be placed in cars, refrigerators, homes, especially when it is not possible to ventilate with clean air, operating theatres in hospitals, bunkers to prevent radioactive contamination.

From a structural point of view, purification and sanitization devices are conventionally composed of a fan capable of sucking in the indoor air and passing it through at least one air filter designed to purify the air before it can be expelled into the environment.

The devices can therefore be equipped with an ionizer to ionize the air, room fragrances, a special ultraviolet (UV) lamp to release ozone, a special ultraviolet (UV) lamp to release hydroxyl radicals (OH), molecules with an oxidizing potential higher than ozone but completely safe for humans and living beings, a special ultraviolet (UV) lamp fitted inside a module where photocatalytic material is present, in order to activate the photocatalysis process, HEPA filter to retain fine dust, including pm10.

The known technique described includes some main disadvantages.

In particular, devices of the known art have just one function, i.e. purifying the air and cannot be used for other purposes.

Moreover, devices of the known art do not have a high efficiency which, in case of particularly critical or contaminated environments, can lead to incorrect air purification.

In this situation, the technical task underlying the present invention is to devise a purification and sanitization device capable of substantially overcoming at least part of the mentioned disadvantages.

Within the scope of said technical task, an important aim of the invention is to obtain a purification and sanitization device that is multifunctional and allows for the purification of both air and a solid surface when used for that purpose.

Another important purpose of the invention is to achieve a purification and sanitization device that is highly efficient.

Therefore, a further task of the invention is to create a device to purify and sanitize that ensures the cleansing of the environment in which it is used.

The technical task and the specified purposes are achieved by a purifying and sanitizing device as claimed in the attached claim 1.

Preferred technical solutions are highlighted in the dependent claims.

The features and advantages of the invention are made more clear by the detailed description of preferred embodiments of the invention, with reference to the attached drawings, wherein:

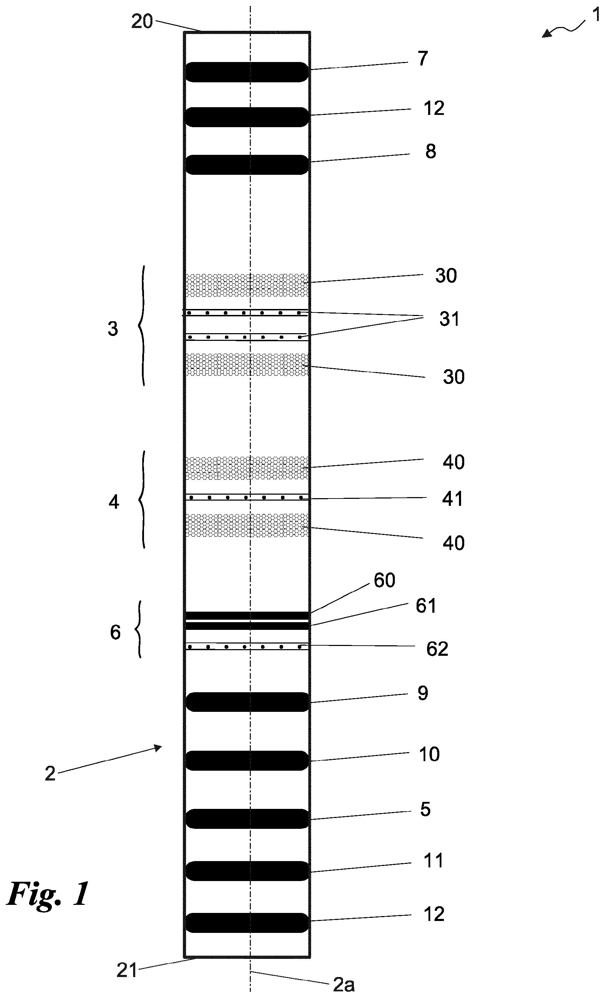

Fig. 1 shows a simplified diagram of a purifying and sanitizing device according to the invention.

In this document, measures, values, shapes, and geometric references (such as perpendicularity and parallelism), when associated with words like "approximately" or other similar terms such as "almost" or "substantially", are to be understood as allowing measurement errors or inaccuracies due to production and/or manufacturing errors and, above all, allowing a slight deviation from the value, measure, shape, or geometric reference to which it is associated. For example, such terms, if associated with a value, preferably indicate a deviation not exceeding 10% of the value itself.

Furthermore, when used, terms like "first", "second", "upper", "lower", "main", and "secondary" do not necessarily identify an order, a relationship priority, or relative position, but can simply be used to more clearly distinguish different components from each other.

Unless otherwise specified, as it results from the following discussions, it is considered that terms like "treatment", "computing", "determination", "calculation", or similar, refer to the action and/or processes of a computer or similar electronic computing device that manipulates and/or transforms data represented as physical, such as electronic quantities in computer system registers and/or memories of, other data similarly represented as physical quantities within computer systems, registers, or other storage, transmission, or display devices.

The measurements and data within this text are to be considered, unless otherwise indicated, as made in International Standard Atmosphere ICAO (ISO 2533:1975). With reference to the Figures, the purification and sanitization device according to the invention is globally denoted by number 1.

The purification and sanitization device 1 is substantially designed to allow the purification and sanitization of at least air. Moreover, the device 1 can also be suitable for the purification of surfaces, as will be explained later, by the use of purified air.

Device 1, therefore, comprises, in broad outline, at least one duct 2.

The duct 2 is substantially a closed tubular element, preferably equipped with two inlets. Therefore, the duct 2 develops along a continuous path preferably determined by a development trajectory 2a.

Furthermore, having two inlets, the duct 2 comprises an inlet 20 and an outlet 21. Thus, the duct 2 develops along the development trajectory 2a between the inlet 20 and the outlet 21.

Preferably, the inlet 20 is designed to allow the entry of air into the duct 2 from an external environment. The outlet 21, on the other hand, is designed to allow the exit of air from the duct 2 towards an external environment.

Naturally, inlet 20 and outlet 21 are arranged at opposite ends of the duct 2. Moreover, the environments with which inlet 20 and outlet 21 interact can be the same environment, or different environments.

For example, outlet 21 may communicate with a closed external environment, for example, a room, and the inlet 20 may communicate with an open external environment, for example, the atmospheric environment outside a room.

The device 1, therefore, also includes a first stage 3.

The first stage 3 is a photocatalytic stage, that is designed to define photocatalysis reactions.

Photocatalysis, as is known, is a reaction by which, when a semiconductor crystal is irradiated with light of sufficiently high energy, an electron from the valence band can absorb the energy of the photon and move to the conduction band, leaving a gap in the valence band.

The electrons and gaps thus generated then migrate to the surface of the crystal, where they react with the absorbed species, as electrons acceptors or donors. The products formed following this reaction are typically free radicals, highly reactive, which generally then attack other components of the system, such as pollutants, viruses, bacteria, or other substances.

In particular, to achieve photocatalysis, the first stage 3 comprises at least a first barrier 30 and a first emitter 31.

The first barrier 30 is preferably made of porous nano- or micro-ceramics. Moreover, the first plate is preferably enriched with titanium dioxide and apatite. In addition, the first barrier 30 can be further enriched with silver and copper ions.

Apatite, due to its crystalline structure, promotes a greater production of hydrogen peroxide while silver and copper ions have an antiseptic function, already upon contact in the presence of humidity in the air.

Therefore, the first barrier 30 is placed in the duct 2 transversely to the development trajectory 2a.

More in detail, for example, the first barrier 30 can be a honeycomb plate. The channels of the honeycomb are, therefore, preferably parallel to the development trajectory 2a.

Substantially, the first barrier 30 defines an obstacle through which the air passes by, through the pores, to be able to proceed inside the duct 2.

The first emitter 31 is a light emitter. In detail, the first emitter 31 is configured to emit a first UV-C light. In particular, the first emitter 31 emits light so that the first light can hit the first barrier 30.

Furthermore, the first emitter 31 is configured to vary, on command, the wavelength of the first light at least between 250 nm and 390 nm with a tolerance of ± 10 nm.

Even more in detail, the first emitter 31 is configured to be able to emit at least first lights at specific wavelengths equal to 254 nm, 280 nm, and 380 nm with a tolerance of ± 10 nm.

Moreover, the first emitter 31 may include a plurality of sources, preferably of LED type.

For example, the first emitter 31 may include one or more strips of sources wherein the sources are alternated.

In particular, the sources can be seven in number and alternated, for example, as follows: the sources can alternate from 0.5 to 1 W and can respectively include LED with a lens up to 180 degrees, with different wavelengths of 254 +\- 5 nm with virucidal, bactericidal, fungicidal, and sporicidal function, activating a minimum photocatalysis; an additional LED with wavelengths of 280 +\- 10 nm which are also virucidal, bactericidal, sporicidal, and fungicidal with a power of photocatalysis activation greater than the first ones and a further LED with a wavelength of 380 +\-10 nm also have biocidal action but represent the ideal wavelength for maximum activation of photocatalysis.

Therefore, each strip may include 3 LEDs of 254 +\- 5 nm, two LEDs of 280 +\- 10 nm, and two LEDs of 380 +\-10 nm, each approximately of 0.5/1 W of LED light, each W of LED light corresponds to about 7 W of light power produced.

In this way, a light power is produced that guarantees the inactivation of DNA and RNA of viruses and bacteria.

In the preferred embodiment of the invention, the first stage 3 comprises two first barriers 30 between which the first emitter 31 is preferably arranged, comprising a plurality of strips, in particular four, mutually opposed.

The device 1 advantageously also includes a second stage 4.

The second stage 4, similarly to the first stage 3, is of the photocatalytic type, that is, designed to define photocatalysis reactions.

Therefore, the second stage 4 also includes at least a second barrier 40 and a second emitter 41.

The second barrier 40 can also be made of nano- or micro-ceramics. In any case, preferably, the second barrier 40 is still porous. Moreover, the second barrier 40 is preferably enriched with tungsten trioxide.

Therefore, the second barrier 40 is also placed in the duct 2 transversely to the development trajectory 2a.

More in detail, for example, the second barrier 40 can be a honeycomb plate. The channels of the honeycomb are, therefore, preferably parallel to the development trajectory 2a. Moreover, the honeycomb plate can be semi-circular to be irradiated by light even inside the honeycombs thus to enhance the production of H₂O₂ and exploit their whole surface.

Substantially, the second barrier 40 defines an obstacle through which the air passes by, through the pores, to be able to proceed inside the duct 2.

The second emitter 41 is a light emitter. In detail, the second emitter 41 is configured to emit a second visible light. In particular, the second emitter 41 emits light in such a way that the second light can hit the second barrier 40.

Furthermore, the second emitter 31 is configured to vary, on command, the wavelength of the first light at least between 390 nm and 760 nm with a tolerance of ± 10 nm.

Even more in detail, the second emitter 41 is configured to be able to emit at least first lights at specific wavelengths equal to 500 nm, 600 nm, and 700 nm with a tolerance of ± 10 nm.

Moreover, the second emitter 41 may include a plurality of sources, preferably of LED type.

For example, the second emitter 41 may include one or more strips of sources in which the sources are alternated.

In particular, the sources can be seven in number and alternated, for example, as follows: the sources can alternate from 0.5 to 1 W and can respectively include LED with a lens up to 180 degrees, with different wavelengths of 500 +\- 5 nm with virucidal, bactericidal, fungicidal, and sporicidal function, activating a minimum photocatalysis; an additional LED with wavelengths of 600 +\- 10 nm also having a virucidal, bactericidal, sporicidal, and fungicidal function with a power of photocatalysis activation greater than the first ones and a further LED with a wavelength of 700 +\- 10 nm also having biocidal action but representing the ideal wavelength for maximum activation of photocatalysis.

Therefore, each strip may include 3 LEDs of 500 +\- 5 nm, two LEDs of 600 +\- 10 nm, and two LEDs of 700 +\-10 nm each of about 0.5/1 W of LED light each W of LED light corresponding to about 7 W of light power produced.

In the preferred embodiment of the invention, the second stage 4 comprises two second barriers 40 between which the first emitter 31 is preferably arranged, comprising a composition of more sources, for example 7 alternating LEDs, with wavelengths of 500-600-700 +\- 10 nm positioned to irradiate tungsten trioxide to enhance the production of H₂O₂.

Advantageously, the device 1 also includes a nebulizer 5.

The nebulizer 5 is substantially configured to produce aerosols of nanometric microparticles. In particular, it is configured to manipulate a liquid substance with virucidal, bactericidal, sporicidal, and fungicidal power from a source part of the device.

Thus, the nebulizer 5 is configured to introduce the aerosol of microparticles into the duct 2 between the second plate 40 and the outlet 21.

The nebulizer 5 is therefore configured to allow mixing of air and aerosol so as to be able to expel the mixture from the outlet 21 and allow for the sanitization of an environment, even in the presence of people, as well as a surface.

The device 1 could therefore include a processor. The latter could be operationally connected to the nebulizer 5 and configured to allow its activation and adjustment with reference to liquid flow rate.

Moreover, the processor could be remotely controllable. In such case, the nebulizer 5 could be remotely programmable, through the processor, in particular for example via an electronic application, thus allowing the adjustment of the liquid flow rate to be converted into aerosol and dispensing and determining the cyclic working and resting times according to the needs of the environment and the number of people involved.

The importance of the nebulizer 5 is, in general, given by the fact of being able to sanitize air and surfaces, using a chemical composition that deposits on all surfaces and crevices, as well as hydrogen peroxide, and penetrates even into fabrics up to, for example, 5mm.

The device 1 may also include a first filter 6, in addition to what has already been described.

The first filter 6 is placed in the duct 2 preferably between the second barrier 40 and the nebulizer 5. Furthermore, the first filter 6 preferably includes a sandwich structure. The latter comprising at least a first layer 60, a second layer 61, and a third emitter 62.

The first layer 60 preferably includes nano-ceramic foam.

The second layer 61, instead, includes titanium dioxide foam.

One or more of the layers 60, 61 is furthermore preferably enriched with silver and copper ions so as to increase the biocidal function.

Therefore, the third light emitter 62 is configured to emit a third light of UV-C or UV-A type configured to strike the layers 60, 61.

The first filter 6, for how it is structured, has substantially a passive and bioactive filtration function.

In particular, the first filter 6 has a biocidal function and creates a third photocatalytic stage given that, when subjected to the light from the third emitter 52, the layers 60, 61 act as a micro-mechanical filter and silver and copper are strongly biocidal against all microorganisms including viruses and bacteria.

The device 1 may also include a second filter 7.

If present, the second filter 7 is placed in the duct 2 between the inlet 20 and the first barrier 30. Preferably, the second filter includes activated carbons enriched with silver ions and potassium permanganate and copper. In detail, potassium permanganate helps to decay ozone after use or if present in the environment.

The device 1 may also include a third filter 8.

If present, the third filter 8 is placed in the duct 2 between the inlet 20 and the first barrier 30. Moreover, if the second filter 7 is present, the third filter 8 is preferably placed between the second filter 7 and the first barrier 30.

Therefore, the third filter 8 is of the electrostatic type.

The electrostatic filter essentially ionizes the air passing through electrodes that charge the particles and microorganisms such as viruses, bacteria, spores, fungi. Therefore, when the particles of microorganisms pass through the collection section, they are captured by the electric field generated so that the third filter attracts microorganisms towards its surface. Exiting the third filter 8, therefore, there is an air flow free of contaminants, being highly efficient and effective also in the destruction of VOCs, as well as in the disintegration of viruses and bacteria.

The third filter 8 could, therefore, also be equipped with a collection drawer for periodic cleaning.

The device 1 may also include an ionizer 9.

The ionizer 9 is preferably placed between the second barrier 40 and the nebulizer 5. Therefore, if the first filter 6 is present, the ionizer 9 can be placed, in particular, between the first filter 6 and the nebulizer 5.

The ionizer 9 preferably produces negative ions from 200 million/cm³ to 500 million/cm³. Essentially, the ions eliminate molds, spores, allergens, bacteria, viruses, and particularly disintegrate the membranes of viruses and bacteria.

The device 1 may include, in addition, a disruptor 10. The disruptor 10, if present, is preferably placed in the duct 2 between the second barrier 40 and the nebulizer 5. If the ionizer 9 is present, the disruptor 10 is placed between the ionizer 9 and the nebulizer 5.

Therefore, the disruptor 10 is preferably a cold plasma disruptor and is configured to disintegrate viruses and bacteria.

The device 1 may also include an ozonizer 11.

The ozonizer 11 is preferably placed in the duct 2 between the nebulizer 5 and the outlet 21.

Therefore, the ozonizer 11 can be operationally connected to the processor. The latter can be configured to turn on or off, on command, the ozonizer 11.

The device 1 might, moreover, include an apparatus 12.

If present, the apparatus 12 is configured to convey air from the inlet 20 towards the outlet 21. In this regard, the apparatus 12 can be placed in the duct 2 between the inlet 20 and the first stage 3.

The apparatus 12 can essentially include a fan. Therefore, the apparatus 12 can fulfill the main suction function.

For example, the volumetric flow rate of the apparatus 12 can be between 200 m³/h and 800 m³/h.

Moreover, it is preferable that the inlet 20 is placed in an area of the device 1 with a lower gravitational potential compared to the area where the outlet 21 is positioned. This means that, if the device 1 rests on a floor, the inlet 20 can be positioned in a lower area, than the floor of the device 1, while the outlet 21 can be positioned on an upper area.

In addition, the device 1 can include another apparatus 12.

The latter can then be placed between the nebulizer 5 and the outlet 21.

Moreover, the additional apparatus 12 can be configured to allow air exchange with controlled mechanical ventilation. It can therefore include at least a cylinder that at the outlet 21 communicates with a room, through a calibrated opening for example of a wall that leads to the outside of a room. To allow adjustments, the apparatus 12 can also be telescopic and of various sizes.

Even more in detail, half of it can be equipped with a fan that introduces pure and clean air from the outside to the inside and the other half emits air always through a fan from the inside to the outside.

Between apparatus 12 and outlet 21, there can also be present other filters like a mechanical dust filter, an activated carbon filter enriched with silver ions. If these filters are present, it is possible to obtain a first passive filtration and a second bioactive one. Therefore, at the outlet 21, copper heat exchangers can also be present to ensure controlled air exchange.

The device 1 can also be equipped with sensors.

In detail, the device 1 can further comprise one or more first sensors.

If present, the first sensors are placed in the duct 2. Therefore, they are configured to detect at least the presence of viruses or bacteria inside the duct 2.

Therefore, the processor can also be operationally connected to the one or more sensors. Moreover, the processor can also be operationally connected to emitters 31, 41, and to said nebulizer 5. Therefore, the processor can be configured to operate the emitters 31, 41, and/or the nebulizer in response to a virus or bacteria detection signal generated by said one or more first sensors.

The device 1 may also include one or more second sensors. If present, said one or more second sensors are placed at the outlet 21.

Therefore, the second sensors can be configured to detect at least the presence of users at the outlet 21.

The processor can then also be operationally connected to the one or more second sensors. Therefore, the processor can also be configured to operate the ozonizer 11 exclusively when the one or more second sensors do not detect the presence of users.

The sensors might include also one or more among a CO₂ detector sensor, important for its saturation, a contagion vehicle, a temperature detector sensor, a humidity detector sensor, a gas detector sensor, and sensors for detecting 1.0-2.5-10.0-0.1MP.

As already mentioned, the processor can, in any case, remotely interface with device 1 management systems.

The operation of device 1 previously described in structural terms is substantially similar to any known air sanitization device.

However, the device 1 according to the invention achieves important advantages. Indeed, the purification and sanitization device 1 is multifunctional and allows to purify both air and a solid surface when used for this purpose.

Moreover, the purification and sanitization device 1 is highly efficient.

In conclusion, the device 1 for purifying and sanitizing ensures the cleanliness of the environment in which it is used.

In addition, thanks to its simplified structure, device 1 is mobile, versatile, and can be placed anywhere. Therefore, device 1 is very useful in hospital environments to combat nosocomial infections which, as is known, are the cause of cross-infections and sepsis with high mortality.

Furthermore, the versatility of device 1 makes it suitable for use in schools, nurseries, public places, nursing homes, and even as a wall device, for example, similar to a split for air conditioners.

## Claims

1. Purification and sanitization device (1), said purification and sanitization device (1) comprising:
- a duct (2) developing along a development trajectory (2a) between an inlet (20) designed to allow the entry into said duct (2) of air from an external environment and an outlet (21) designed to allow the exit of said air from said duct (2) towards an external environment;
- a first photocatalytic stage (3) including:
- a first barrier (30) made of porous nano- or micro-ceramics enriched with titanium dioxide and apatite and placed in said duct (2) transversely to said development trajectory (2a),
- a first light emitter (31) configured to emit a first UV-C light in such a way that said first light can strike said first barrier (30) and to vary, on command, the wavelength of said first light at least between 250 nm and 390 nm with a tolerance of ± 10 nm; and **characterized in that** said purification and sanitization device (1) further comprises
- a second photocatalytic stage (4) including:
- a second porous barrier (40) made of tungsten trioxide and placed in said duct (2) between said first barrier (30) and said outlet (21) transversely to said development trajectory (2a),
- a second light emitter (41) configured to emit a second visible light to strike said second barrier (40) and to vary, on command, the wavelength of said first light at least between 390 nm and 760 nm with a tolerance of ± 10 nm; - a source of liquid substance with virucidal, bactericidal, sporicidal and fungicidal power and
- a nebulizer (5) configured to produce aerosols of nanometric microparticles, manipulating the liquid substance with virucidal, bactericidal, sporicidal, and fungicidal power, and introduce them into said duct (2) between said second plate (40) and said outlet (21);

2. Device (1) according to claim 1, further comprising a first filter (6) placed in said duct (2) between said second barrier (40) and said nebulizer (5) and including a sandwich structure comprising at least a first layer (60) in nano-ceramics foam, a second layer (61) in titanium dioxide foam, and a third light emitter (62) configured to emit a third UV-C or UV-A light to strike said layers (60, 61), one or more of said layers (60, 61) being enriched with silver and copper ions.

3. Device (1) according to any of the preceding claims, wherein said purification and sanitization device (1) further comprises a second filter (7) made of activated carbons enriched with silver ions and potassium permanganate placed in said duct (2) between said inlet (20) and said first barrier (30).

4. Device (1) according to any of the preceding claims, wherein said purification and sanitization device (1) further comprises a third electrostatic filter (8) placed in said duct (2) between said inlet (20) and said first barrier (30).

5. Device (1) according to any of the preceding claims, wherein said purification and sanitization device (1) further comprises an ionizer (9) placed in said duct (2) between said first filter (6) and said nebulizer (5).

6. Device (1) according to any of the preceding claims, wherein said purification and sanitization device (1) further comprises a cold plasma disruptor (10) configured to disintegrate viruses and bacteria and placed inside said duct (2) between said second barrier (40) and said nebulizer (5).

7. Device (1) according to any of the preceding claims, wherein said purification and sanitization device (1) further comprises an ozonizer (11) placed in said duct (2) between said nebulizer (5) and said outlet (21).

8. Device (1) according to any of the preceding claims, wherein said purification and sanitization device (1) further comprises an apparatus (12) configured to convey air from said inlet (20) towards said outlet (21) and placed inside said duct (2) at least between said inlet (20) and said first stage (3).

9. Device (1) according to any of the preceding claims, wherein said purification and sanitization device (1) further comprises one or more first sensors placed in said duct (2) and configured to detect at least the presence of viruses or bacteria in said duct (2) and a processor operationally connected to said one or more sensors and at least said emitters (31, 41) and said nebulizer (5) and configured to operate said emitters (31, 41) and/or said nebulizer in response to a signal detecting said viruses or bacteria, generated by said one or more first sensors.

10. Device (1) according to claims 7 and 9, wherein said purification and sanitization device (1) further comprises one or more second sensors placed at said outlet (21) and configured to detect at least the presence of users at said outlet (21) and said processor is operationally connected to said one or more second sensors and configured to operate said ozonizer (11) exclusively when said one or more second sensors do not detect the presence of users.

## Patentansprüche

1. Reinigungs- und Desinfektionsvorrichtung (1), wobei die Reinigungs- und Desinfektionsvorrichtung (1) Folgendes umfasst:
- einen Kanal (2), der sich entlang einer Verlaufsbahn (2a) zwischen einem Einlass (20), der dazu ausgelegt ist, den Eintritt von Luft aus einer äußeren Umgebung in den Kanal (2) zu ermöglichen, und einem Auslass (21), der dazu ausgelegt ist, den Austritt der Luft aus dem Kanal (2) in Richtung einer äußeren Umgebung zu ermöglichen, erstreckt;
- eine erste photokatalytische Stufe (3), umfassend:
- eine erste Barriere (30), die aus porösen Nano- oder Mikrokeramiken besteht, welche mit Titandioxid und Apatit angereichert sind, und die in dem Kanal (2) quer zu der Verlaufsbahn (2a) angeordnet ist,
- einen ersten Lichtemitter (31), der dazu konfiguriert ist, ein erstes UV-C-Licht derart zu emittieren, dass das erste Licht auf die erste Barriere (30) treffen kann, und auf Befehl die Wellenlänge des ersten Lichts zumindest zwischen 250 nm und 390 nm mit einer Toleranz von ± 10 nm zu variieren;
und **dadurch gekennzeichnet, dass** die Reinigungs- und Desinfektionsvorrichtung (1) ferner Folgendes umfasst:
- eine zweite photokatalytische Stufe (4), umfassend:
- eine zweite poröse Barriere (40), die aus Wolframtrioxid besteht und in dem Kanal (2) zwischen der ersten Barriere (30) und dem Auslass (21) quer zu der Verlaufsbahn (2a) angeordnet ist,
- einen zweiten Lichtemitter (41), der dazu konfiguriert ist, ein zweites sichtbares Licht zu emittieren, um auf die zweite Barriere (40) zu treffen, und auf Befehl die Wellenlänge des ersten Lichts zumindest zwischen 390 nm und 760 nm mit einer Toleranz von ± 10 nm zu variieren;
- eine Quelle einer flüssigen Substanz mit viruzider, bakterizider, sporizider und fungizider Wirkung und
- einen Vernebler (5), der dazu konfiguriert ist, Aerosole aus nanometrischen Mikropartikeln unter Verarbeitung der flüssigen Substanz mit viruzider, bakterizider, sporizider und fungizider Wirkung zu erzeugen und diese in den Kanal (2) zwischen der zweiten Platte (40) und dem Auslass (21) einzubringen;

2. Vorrichtung (1) nach Anspruch 1, ferner umfassend einen ersten Filter (6), der in dem Kanal (2) zwischen der zweiten Barriere (40) und dem Vernebler (5) angeordnet ist und eine Sandwichstruktur umfasst, die mindestens eine erste Schicht (60) aus Nanokeramikschaum, eine zweite Schicht (61) aus Titandioxidschaum und einen dritten Lichtemitter (62) umfasst, der dazu konfiguriert ist, ein drittes UV-C- oder UV-A-Licht zu emittieren, um auf die Schichten (60, 61) zu treffen, wobei eine oder mehrere der Schichten (60, 61) mit Silber- und Kupferionen angereichert sind.

3. Vorrichtung (1) nach einem der vorhergehenden Ansprüche, wobei die Reinigungs- und Desinfektionsvorrichtung (1) ferner einen zweiten Filter (7) umfasst, der aus mit Silberionen und Kaliumpermanganat angereicherten Aktivkohlen besteht und in dem Kanal (2) zwischen dem Einlass (20) und der ersten Barriere (30) angeordnet ist.

4. Vorrichtung (1) nach einem der vorhergehenden Ansprüche, wobei die Reinigungs- und Desinfektionsvorrichtung (1) ferner einen dritten elektrostatischen Filter (8) umfasst, der in dem Kanal (2) zwischen dem Einlass (20) und der ersten Barriere (30) angeordnet ist.

5. Vorrichtung (1) nach einem der vorhergehenden Ansprüche, wobei die Reinigungs- und Desinfektionsvorrichtung (1) ferner einen Ionisator (9) umfasst, der in dem Kanal (2) zwischen dem ersten Filter (6) und dem Vernebler (5) angeordnet ist.

6. Vorrichtung (1) nach einem der vorhergehenden Ansprüche, wobei die Reinigungs- und Desinfektionsvorrichtung (1) ferner einen Kaltplasma-Disruptor (10) umfasst, der dazu konfiguriert ist, Viren und Bakterien zu desintegrieren, und der innerhalb des Kanals (2) zwischen der zweiten Barriere (40) und dem Vernebler (5) angeordnet ist.

7. Vorrichtung (1) nach einem der vorhergehenden Ansprüche, wobei die Reinigungs- und Desinfektionsvorrichtung (1) ferner einen Ozonisator (11) umfasst, der in dem Kanal (2) zwischen dem Vernebler (5) und dem Auslass (21) angeordnet ist.

8. Vorrichtung (1) nach einem der vorhergehenden Ansprüche, wobei die Reinigungs- und Desinfektionsvorrichtung (1) ferner eine Einrichtung (12) umfasst, die dazu konfiguriert ist, Luft von dem Einlass (20) in Richtung des Auslasses (21) zu fördern, und die innerhalb des Kanals (2) zumindest zwischen dem Einlass (20) und der ersten Stufe (3) angeordnet ist.

9. Vorrichtung (1) nach einem der vorhergehenden Ansprüche, wobei die Reinigungs- und Desinfektionsvorrichtung (1) ferner einen oder mehrere erste Sensoren, die in dem Kanal (2) angeordnet und dazu konfiguriert sind, zumindest das Vorhandensein von Viren oder Bakterien in dem Kanal (2) zu erfassen, sowie einen Prozessor umfasst, der mit dem einen oder den mehreren Sensoren und zumindest mit den Emittern (31, 41) und dem Vernebler (5) wirkverbunden und dazu konfiguriert ist, die Emitter (31, 41) und/oder den Vernebler als Reaktion auf ein die Viren oder Bakterien erfassendes Signal zu betreiben, das von dem einen oder den mehreren ersten Sensoren erzeugt wird.

10. Vorrichtung (1) nach den Ansprüchen 7 und 9, wobei die Reinigungs- und Desinfektionsvorrichtung (1) ferner einen oder mehrere zweite Sensoren umfasst, die an dem Auslass (21) angeordnet und dazu konfiguriert sind, zumindest das Vorhandensein von Benutzern an dem Auslass (21) zu erfassen, und wobei der Prozessor mit dem einen oder den mehreren zweiten Sensoren wirkverbunden und dazu konfiguriert ist, den Ozonisator (11) ausschließlich dann zu betreiben, wenn der eine oder die mehreren zweiten Sensoren das Vorhandensein von Benutzern nicht erfassen.

## Revendications

1. Dispositif de purification et d'assainissement (1), ledit dispositif de purification et d'assainissement (1) comprenant :
- un conduit (2) se développant selon une trajectoire de développement (2a) entre une entrée (20) conçue pour permettre l'entrée dans ledit conduit (2) d'air provenant d'un environnement extérieur et une sortie (21) conçue pour permettre la sortie dudit air dudit conduit (2) vers un environnement extérieur ;
- un première phase photocatalytique (3) comprenant :
- une première barrière (30) constituée de nano- ou micro-céramiques poreuses enrichies en dioxyde de titane et apatite et placée dans ledit conduit (2) transversalement à ladite trajectoire de développement (2a),
- un premier émetteur de lumière (31) configuré pour émettre une première lumière UV-C de manière que ladite première lumière puisse frapper ladite première barrière (30) et pour faire varier, sur commande, la longueur d'onde de ladite première lumière au moins entre 250 nm et 390 nm avec une tolérance de ± 10 nm ;
et **caractérisé en ce que** ledit dispositif de purification et d'assainissement (1) comprend en outre
- une seconde phase photocatalytique (4) comprenant :
- une seconde barrière poreuse (40) constituée de trioxyde de tungstène et placée dans ledit conduit (2) entre ladite première barrière (30) et ladite sortie (21) transversalement à ladite trajectoire de développement (2a),
- un second émetteur de lumière (41) configuré pour émettre une seconde lumière visible pour frapper ladite seconde barrière (40) et pour faire varier, sur commande, la longueur d'onde de ladite première lumière au moins entre 390 nm et 760 nm avec une tolérance de ± 10 nm ; - une source de substance liquide ayant un pouvoir virucide, bactéricide, sporicide et fongicide et
- un nébuliseur (5) configuré pour produire des aérosols de microparticules nanométriques, en manipulant la substance liquide ayant un pouvoir virucide, bactéricide, sporicide et fongicide et les introduire dans ledit conduit (2) entre ladite seconde plaque (40) et ladite sortie (21) ;

2. Dispositif (1) selon la revendication 1, comprenant en outre un premier filtre (6) placé dans ledit conduit (2) entre ladite seconde barrière (40) et ledit nébuliseur (5) et comprenant une structure en sandwich comprenant au moins une première couche (60) en mousse nano-céramique, une seconde couche (61) en mousse de dioxyde de titane, et un troisième émetteur de lumière (62) configuré pour émettre une troisième lumière UV-C ou UV-A pour frapper lesdites couches (60, 61), une ou plusieurs desdites couches (60, 61) étant enrichies en ions argent et cuivre.

3. Dispositif (1) selon l'une quelconque des revendications précédentes, dans lequel ledit dispositif de purification et d'assainissement (1) comprend en outre un second filtre (7) constitué de charbons actifs enrichis en ions argent et permanganate de potassium placé dans ledit conduit (2) entre ladite entrée (20) et ladite première barrière (30).

4. Dispositif (1) selon l'une quelconque des revendications précédentes, dans lequel ledit dispositif de purification et d'assainissement (1) comprend en outre un troisième filtre électrostatique (8) placé dans ledit conduit (2) entre ladite entrée (20) et ladite première barrière (30).

5. Dispositif (1) selon l'une quelconque des revendications précédentes, dans lequel ledit dispositif de purification et d'assainissement (1) comprend en outre un ioniseur (9) placé dans ledit conduit (2) entre ledit premier filtre (6) et ledit nébuliseur (5).

6. Dispositif (1) selon l'une quelconque des revendications précédentes, dans lequel ledit dispositif de purification et d'assainissement (1) comprend en outre un dispositif de rupture à plasma froid (10) configuré pour désintégrer les virus et les bactéries et placé à l'intérieur dudit conduit (2) entre ladite seconde barrière (40) et ledit nébuliseur (5).

7. Dispositif (1) selon l'une quelconque des revendications précédentes, dans lequel ledit dispositif de purification et d'assainissement (1) comprend en outre un ozoniseur (11) placé dans ledit conduit (2) entre ledit nébuliseur (5) et ladite sortie (21).

8. Dispositif (1) selon l'une quelconque des revendications précédentes, dans lequel ledit dispositif de purification et d'assainissement (1) comprend en outre un appareil (12) configuré pour acheminer l'air depuis ladite entrée (20) vers ladite sortie (21) et placé à l'intérieur dudit conduit (2) au moins entre ladite entrée (20) et ledit premier étage (3).

9. Dispositif (1) selon l'une quelconque des revendications précédentes, dans lequel ledit dispositif de purification et d'assainissement (1) comprend en outre un ou plusieurs premiers capteurs placés dans ledit conduit (2) et configurés pour détecter au moins la présence de virus ou de bactéries dans ledit conduit (2) et un processeur connecté fonctionnellement audit un ou plusieurs capteurs et au moins auxdits émetteurs (31, 41) et audit nébuliseur (5) et configuré pour faire fonctionner lesdits émetteurs (31, 41) et/ou ledit nébuliseur en réponse à un signal détectant lesdits virus ou bactéries, généré par ledit un ou plusieurs premiers capteurs.

10. Dispositif (1) selon les revendications 7 et 9, où ledit dispositif de purification et d'assainissement (1) comprend en outre un ou plusieurs seconds capteurs placés au niveau de ladite sortie (21) et configurés pour détecter au moins la présence d'utilisateurs au niveau de ladite sortie (21)et ledit processeur est connecté fonctionnellement audit un ou plusieurs seconds capteurs et configuré pour faire fonctionner ledit ozoniseur (11) exclusivement lorsque ledit un ou plusieurs seconds capteurs ne détectent pas la présence d'utilisateurs.
